# EUROPEAN PATENT APPLICATION

(11) **EP 2 112 271 A1**
(43) Date de publication de la demande: **28.10.2009**
(21) Numéro de dépôt: 08290311.3
(22) Date de dépôt: 31.03.2008
(51) Int. Cl.: D06N 7/00, C08J 7/04, A61L 31/04, A61L 31/10

(54) **Association d'un flim et d'un hydrogel a base de chitosane et ses applications en chirurgie**

(71) Demandeur: Analytic Biosurgical Solutions - ABISS, 42000 Saint Etienne (FR)
(72) Inventeur: Ducreux, Laure, 42000 Saint Etienne (FR); Beraud, Jean-Marc, 42000 Saint Etienne (FR)
(74) Mandataire: Sellin, Carole

(57) **Abrégé**

La présente invention concerne une composition comprenant l'association d'un film à base de chitosane, comprenant moins de 20% d'eau (en masse), et d'un hydrogel à base de chitosane, d'un dérivé de chitosane ou d'un complexe de chitosane, comprenant au moins 80% d'eau (en masse). La présente invention concerne également une composition comprenant l'association d'un support textile; d'un film à base de chitosane, comprenant moins de 20% d'eau (en masse) et d'un hydrogel à base de chitosane, d'un dérivé de chitosane ou d'un complexe de chitosane, comprenant au moins 80% d'eau (en masse), tel que l'hydrogel ne soit présent que sur l'une des deux faces du support textile.

Lesdites compositions ont des applications notamment dans l'élaboration de prothèses en vue d'une implantation chirurgicale ou cosméto-esthétique.

La présente invention concerne également des procédés d'obtention desdites compositions.

## Description

La présente demande a trait aux matériaux ayant des qualités de renfort et partiellement biodégradables, utilisés tout particulièrement comme prothèse. Plus particulièrement, la présente demande concerne un composition comprenant l'association d'un film et d'un hydrogel à base de chitosane, elle concerne également un support textile comportant sur l'une de ses faces un film et un hydrogel à base de chitosane, tel que l'hydrogel ne soit présent que sur l'une des deux faces du matériau.

En effet, dans le domaine des dispositifs médicaux ou chirurgicaux implantables, il est souhaitable d'avoir recours à des biomatérieux d'origine naturelle, qui soient biocompatibles, biorésorbables et bioassimilables par l'organisme. De plus, des matériaux pour une telle utilisation doivent idéalement également prévenir les adhésions post-opératoires, notamment à l'issue des interventions chirurgicales abdomino-thoraciques et pelviennes.

En réponse à ce besoin d'un matériau composite qui puisse à la fois jouer un rôle de renfort et éventuellement de barrière physique mécanique entre les tissus, et qui ait des propriétés anti-adhérentielles au cours de la cicatrisation, il a été proposé essentiellement des membranes anti-adhérentielles à base d'acide hyaluronique, de collagène ou de cellulose. Toutefois, il est mentionné dans la littérature que ces matériaux, quand ils sont utilisés pour la prévention des adhérences post-chirurgicales, ne donnent pas entièrement satisfaction, notamment pour les raisons suivantes :
- certains de ces matériaux ont une toxicité résiduelle (notamment dans le cas d'une réticulation chimique à base d'aldéhydes) ;
- la biodégradabilité n'est pas suffisamment contrôlable pour pouvoir être mise en adéquation avec le maintien de leur fonction de barrière physique entre les tissus ;
- la résistance mécanique de certains de ces matériaux est trop faible et ils ne peuvent résister aux tensions imposées après implantation ;
- pour les matériaux à base de collagène, ils ne peuvent pas être mis sous forme de solutions filtrables sur des filtres, dont la porosité est suffisamment faible pour permettre la rétention de contaminants biologiques.

Un autre polymère qui a été utilisé afin de créer des biomatériaux est le chitosane. Il s'agit d'un biopolymère qui est décrit comme biocompatible, biorésorbable et bioactif. En effet, d'après la littérature, le chitosane est connu comme possédant les propriétés biologiques suivantes : biodégradable, biocompatible, non-toxique, hémocompatible, cytocompatible, hémostatique, effet cicatrisant, activités bactériostatiques et fongistatiques.

De plus, le chitosane favorise l'adhésion et la prolifération cellulaire et il est connu pour sa biostimulation dans le processus de reconstitution des tissus. Par ailleurs, il n'est rapporté aucune infection ou allergie liée au chitosane.

Il est également possible de faire varier le temps de résorption du chitosane, en jouant sur ses propriétés physico-chimiques.

Par ailleurs, le chitosane est d'une d'utilisation relativement facile et il peut être produit sous différentes formes physiques, notamment des solutions, des films, des fibres, des hydrogels, des microparticules.

Cependant, bien que possédant les propriétés biologiques souhaitées pour une utilisation dans des implants chirurgicaux, sa mise en oeuvre a montré certaines difficultés.

La demande de brevet FR2 848 118 par exemple propose une prothèse composite comprenant un matériau textile de renfort associé à un hydrogel de chitosane. Cependant, un inconvénient important de cette réalisation est que l'hydrogel, bien que déposé sur une seule des deux faces du matériau textile, a tendance à envahir les deux faces. En effet, il est généralement souhaitable dans ce domaine d'utiliser des tricots fins à larges pores, afin de limiter l'introduction de corps étrangers dans le corps. Or dans le cas de tels tricots, l'hydrogel a tendance à entièrement pénétrer dans les pores du tricot et se trouve donc sur les deux faces du tricot. Ceci est un important inconvénient dans le cas par exemple du traitement des éventrations où il est essentiel que la face du tricot en contact avec les viscères empêche toute colonisation cellulaire. L'utilisation de tricots plus épais ou avec des pores plus fins n'est pas souhaitable en vue d'une implantation chirurgicale.

La demande de brevet FR 2 837 829 décrit quant à elle un support à base de fibres organiques et/ou inorganiques recouvert sur au moins une de ses faces par un film de chitosane, obtenu par enduction. Un inconvénient important de cette réalisation est la masse trop faible de chitosane déposée. De plus, il ne s'agit pas d'un hydrogel, mais d'un film dont les propriétés sont moins avantageuses en terme d'obstacle aux adhérences post-opératoires.

Un problème que se propose de résoudre la présente invention est donc la réalisation d'un support textile qui soit recouvert sur l'une de ses faces uniquement d'un hydrogel à base de chitosane. Une telle composition est obtenue en associant un film à base de chitosane, et un hydrogel à base de chitosane, d'un dérivé de chitosane ou d'un complexe de chitosane.

Dans le cadre de cette description, les termes qui suivent ont plus particulièrement la signification suivante :
Un **matériau composite** est à prendre dans son sens le plus large comme couvrant toute association de différentes substances naturelles ou articfielles, l'aspect composite pouvant venir de l'association de différentes couches de substances différentes.
Par **chitosane,** on entend un polysaccharide composé de la distribution aléatoire de D-glucosamine liée en β-(1-4) (unité désacétylée) et de N-acétyl-D-glucosamine (unité acétylée). Il peut être produit par désacétylation chimique (en milieu alcalin) ou enzymatique de la chitine ; il existe également à l'état naturel.
Par **dérivé du chitosane,** on désigne tout polymère de chitosane ayant subi une réaction visant à modifier les groupements chimiques du chitosane pour en changer les fonctionnalités, par exemple méthyation, halogénation, ....
Le **degré d'acétylation** (DA) est le pourcentage d'unités acétylées par rapport au nombre d'unités totales, il peut être déterminé par spectrométrie infrarouge à transformée de Fourier (IR-TF) ou par RMN du proton.
Par **béta-glucan (ou β-glucan)** on désigne le β-(1,3)-glucan, le β-(1,4)-glucan, le β-(1,6) glucan ainsi que tout mélange de ces différents types.
Par **hydrogel,** on entend un gel obtenu en milieu aqueux.
Par film ou hydrogel **à base de** X, on entend que le constituant majoritaire (donc à plus de 50%) du film ou de l'hydrogel (en masse) est le composé X, abstraction faite de l'eau. Par exemple, un hydrogel à base de chitosane comprenant au moins 85% d'eau signifie que la masse de chitosane représente plus de la moitié de la masse de l'hydrogel « sec » (c'est-à-dire après soustraction de la masse de l'eau).
Par **milieu aqueux** (ou solution aqueuse), on entend une phase liquide contenant plusieurs espèces chimiques, dont l'eau (H₂O) est le composant ultramajoritaire (solvant), les autres espèces étant ultraminoritaires (solutés).

Selon un premier aspect, la présente invention concerne donc une composition comprenant l'association d'un film à base de chitosane et d'un hydrogel à base de chitosane, d'un dérivé de chitosane ou d'un complexe de chitosane.

Un film à base de chitosane selon l'invention comprend moins de 20% d'eau en masse, de préférence moins de 15%. Selon une variante préférée, un film à base de chitosane, comprend moins de 10% voire moins de 5% d'eau en masse. De préférence, il comprend toutefois au moins 3% d'eau.

Un hydrogel à base de chitosane selon l'invention, ou bien à base d'un dérivé ou d'un complexe de chitosane, comprend au moins 80% d'eau en masse. De préférence, il comprend au moins 90%, voire au moins 95% d'eau en masse. L'hydrogel comprend toutefois de préférence moins de 99% d'eau.

Selon un second aspect, la présente invention propose une composition comprenant l'association d'un support textile, d'un film à base de chitosane et d'un hydrogel à base de chitosane, d'un dérivé de chitosane ou d'un complexe de chitosane.

Comme pour le premier aspect de l'invention, un film à base de chitosane s'entend d'un film comprenant moins de 20% d'eau en masse, de préférence moins de 15%, voire moins de 10 ou 5%. Concernant l'hydrogel à base de chitosane, ou bien à base d'un dérivé ou d'un complexe de chitosane, comme pour le premier aspect, il s'agit selon l'invention d'un hydrogel comprenant au moins 80% d'eau en masse, voire au moins 90% ou au moins 95% d'eau en masse.

Un hydrogel à base de chitosane, d'un dérivé ou d'un complexe de celui-ci selon la premier ou le second aspect de l'invention est un hydrogel insoluble en milieu aqueux aux pHs physiologiques. Les pHs dits physiologiques (ou biologiques) sont les pHs compris entre 5 et 8, et plus généralement compris entre 6 et 7.

Des dérivés de chitosane particulièrement préférés sont par exemple le carboxyméthyl chitosane et le N,O-carboxyméthyl chitosane.

Des complexes de chitosane utilisables dans le cadre de la présente invention sont notamment du xanthane de chitosane, de l'alginate de chitosane, de la carboxyméthylcellulose de chitosane ou de la cellulose de chitosane.

Le dérivé ou le complexe peut être choisi par l'homme du métier en fonction des propriétés recherchées pour l'hydrogel, notamment certaines propriétés biologiques. Il peut également être choisi pour ses propriétés physico-chimiques, notamment si l'on souhaite modifier les conditions du procédé de gélification, ou bien utiliser différents procédés de gélification pour l'hydrogel.

Préférentiellement, l'hydrogel entrant dans les compositions de l'invention est obtenu par un procédé d'évaporation en milieu hydro-alcoolique.

Il est à noter qu'un film à base de chitosane, selon les deux aspects de l'invention, est un film continu. Ce film est sec et se présente de préférence sous la forme d'une feuille fine, lisse, transparente et brillante, facilement manipulable. Cependant, son aspect peut être légèrement différent en fonction de sa composition.

Selon le second aspect de l'invention, l'association entre ces trois composants, support textile, film et hydrogel, est telle que l'hydrogel n'est présent que sur l'une des deux faces du support textile. En effet, l'association des trois composants permet d'obtenir un matériau composite ou multicouche présentant sur une face le support textile et sur l'autre face l'hydrogel. Du fait de l'introduction du film, l'hydrogel n'est pas directement en contact avec le support textile et ne s'infiltre donc pas dans les mailles de ce support.

Le support textile dont il est question peut être tout type de support textile, tissé ou bien non tissé, ce support peut également être tricoté. Des supports préférés dans le cadre de la présente invention sont notamment les tricots, de préférence des tricots à larges pores. Avantageusement lesdits supports textiles ou les tricots sont en polymère. Il peut s'agir de tout type de polymère, naturel ou synthétique. Il peut également s'agir de plusieurs polymères, par exemple un tricot fait de plusieurs fils ou filaments constitués par des polymères distincts. Des polymères préférés sont toutefois les polymères synthétiques, tels que le polyester, le polyfluorure de vinylidène (PVDF), et tout particulièrement le polypropylène. Un exemple de support textile est notamment un tricot (ou treillis) à larges pores en monofilaments de polypropylène.

Les fils ou filaments utilisés ont par exemple un diamètre compris entre 0,06 mm et 0,2 mm, de préférence entre 0,08 et 0,15 mm. Il peut s'agir notamment de monofilaments ayant des diamètres différents, par exemple un tricot mixte constitué d'un monofilament de 0,15 mm et d'un monofilament de 0,08 mm, de préférence tous les deux en polypropylène.

Il est souhaitable, notamment pour les utilisations comme implants chirurgicaux, que le support textile ne soit pas résorbable *in vivo,* ou bien à une échelle de temps très importante, par exemple après plusieurs années.

Il est également souhaitable, pour les utilisations en chirurgie, que le support textile soit un tricot dit « light » pour éviter d'implanter des quantités importantes de polymère dans le corps et éviter de plus les éventuelles réactions inflammatoires.

De ce fait, des masses surfaciques particulièrement appropriées pour le support textile dans le cadre de l'invention sont comprises entre 10 et 110 g/m², et plus particulièrement entre 22 g/m² et 84 g/m². Quant à l'épaisseur dudit tricot, elle est de préférence comprise entre 0,1 et 1 mm, plus particulièrement entre 0,25 et 0,5 mm.

Le support textile utilisé dans le cadre de l'invention possède de préférence des pores dont la taille est de 200 µm ou plus, dans au moins une des trois directions.

Selon une mise en oeuvre préférée d'une composition de l'invention, le film est déposé sur le support textile par enduction du support avec une solution à base de chitosane. Une telle enduction peut être réalisée par différentes techniques bien connues de l'homme du métier.

De préférence, l'enduction est réalisée par l'immersion du support textile dans une solution à base de chitosane. Le support textile peut notamment être déposé à la surface d'une solution à base de chitosane étalée sur un support. Il est également envisageable inversement de recouvrir le support textile avec ladite solution à base de chitosane.

Un exemple de réalisation d'une telle enduction est décrit dans la section expérimentale de la demande.

Lors de la réalisation du film par enduction du support avec une solution à base de chitosane, on utilise de préférence une solution ayant une viscosité suffisamment importante pour combler les pores du textile. On peut faire varier la viscosité en modifiant la concentration en chitosane ou en modifiant la masse molaire du chitosane utilisé. De préférence, la concentration en chitosane de la solution utilisée est comprise entre 1% et 2,5% [w/w] pour des chitosane à hautes masses molaires moyennes et entre 2,5 % et 4% [w/w]. pour des chitosane à faibles masses molaires moyennes.

Une composition selon le premier ou le second aspect de l'invention comprend de préférence un film dont l'épaisseur est inférieure à 0,15 mm, voire de préférence inférieure à 0,1 mm. Selon un mode de réalisation tout particulièrement préféré, le film a une épaisseur encore inférieure, de préférence inférieure à 0,03 mm.

Quant à l'épaisseur de l'hydrogel dans les compositions selon l'invention, elle peut varier selon les applications envisagées et être par exemple de l'ordre du millimètre, par exemple inférieure à 2mm, ou bien inférieure à 1,5 mm, par exemple entre 0,5 et 1 mm. L'épaisseur de l'hydrogel est de préférence supérieure à celle du film dans les compositions de l'invention.

Dans une composition selon l'un quelconque des aspects de l'invention, il est à noter que l'association entre le film à base de chitosane et l'hydrogel à base de chitosane, d'un dérivé ou d'un complexe de celui-ci, est une association stable et non une superposition de deux couches. Il est primordial de pouvoir manipuler les compositions de l'invention sans qu'il n'y ait de désolidarisation du film et de l'hydrogel. De même, l'association du film et de l'hydrogel doit également être stable dans le temps et stable en milieu aqueux aux pHs biologiques ou physiologiques. Dans les applications envisagées comme prothèse, il est en effet essentiel qu'il n'y ait pas de désolidarisation du film et de l'hydrogel après mise en place de la prothèse. De ce fait, dans la présente invention, l'association du film et de l'hydrogel s'entend d'une liaison stable permettant la manipulation, la stérilisation et le maintien de l'association ; il est donc important que le film et l'hydrogel soient accrochés ou liés l'un à l'autre.

Un test permettant de définir si l'association entre un film (avec ou sans support textile) et un hydrogel est stable consiste notamment à manipuler cette association dans de l'eau à des pH physiologiques. S'il n'y a pas de désolidarisation du film et de l'hydrogel, on considère alors que les deux sont suffisamment « accrochés » l'un à l'autre pour former une association ou liaison au sens de la présente invention.

Selon une mise en oeuvre préférée de la présente invention, l'accroche permettant l'association entre le film et l'hydrogel est réalisée par mise en contact d'un film non complètement neutralisé avec l'hydrogel en cours de formation. On obtient ainsi une association stable telle que requise pour les compositions de l'invention.

Par film non complètement neutralisé, on entend que le film à base de chitosane comprend au moins 30% des groupements amine du chitosane qui soient sous forme protonée. De préférence, au moins 50%, voire au moins 60% des groupements amine du chitosane du film sont sous forme -NH₃⁺. Bien entendu, selon la composition du film, et notamment la teneur en chitosane, il peut être avantageux d'utiliser un film dont le nombre de groupement amines sous forme protonée est encore supérieur, par exemple au moins 70 voire 75% des groupement amines du chitosane sont sous forme -NH₃⁺.

Par hydrogel en cours de formation, on entend essentiellement un hydrogel avant le point de gélification. Il n'est pas nécessaire que l'hydrogel soit juste en dessous du point de gélification ; il suffit que l'hydrogel ne soit pas encore formé, il peut s'agir notamment encore d'une solution aqueuse dont la gélification est tout juste initiée, voire non encore initiée ou sur le point d'être initiée.

Il est à noter que dans les compositions selon le premier et le second aspect de l'invention, le film et l'hydrogel peuvent avoir la même composition ou inversement peuvent avoir des compositions différentes (indépendamment de la teneur en eau). Il est notamment envisagé que l'hydrogel soit formé à base d'un dérivé ou d'un complexe de chitosane, le film étant quant à lui à base de chitosane. Il peut également être avantageux au contraire de réaliser une composition telle que le film et l'hydrogel sont tous les deux à base de chitosane, avec ou non des composés additionnels identiques. De préférence, la teneur en chitosane et en composés additionnels n'est pas identique dans le film et dans l'hydrogel.

Le chitosane utilisé dans le cadre de la présente invention peut provenir de différentes sources. En effet, on trouve du chitosane ou de la chitine, permettant d'obtenir du chitosane, dans l'exosquelette des arthropodes (crustacés) et dans l'endosquelette des céphalopodes (notamment calamars), mais aussi dans la paroi des champignons. Selon un aspect particulier de l'invention, le chitosane qui est à la base du film est un chitosane d'origine animale, avec un faible risque d'infectiosité (carapaces de crustacés ou endosquelettes de calamars), végétale, ou non-animale (champignons de Paris, nom botanique : *Agaricus bisporus*) ce qui se révèle un avantage intéressant pour la fabrication de dispositifs médicaux implantables du fait des exigences réglementaires dans ce secteur. De manière préférée, le chitosane utilisé n'est pas d'origine animale, et il est de préférence extrait de champignons, notamment de champignons de Paris.

En effet, dans le cadre des différentes applications médicales envisagées pour les compositions de l'invention, il est particulièrement avantageux de n'avoir aucune matière première qui soit d'origine animale, les exigences en matière de sécurité sanitaire étant particulièrement strictes dans le cas de matière d'origine animale.

De manière similaire, le chitosane, le dérivé ou le complexe de celui-ci, qui est à la base de l'hydrogel dans les compositions de l'invention, est également de préférence d'origine non animale, et de manière particulièrement préférée extrait des champignons, notamment champignons de Paris.

Il peut être particulièrement avantageux que tant le film que l'hydrogel soient tous les deux à base de chitosane dont l'origine n'est pas animale, par exemple extrait de champignons.

Dans le cadre des compositions selon les différents aspects de cette invention, le chitosane dont est constitué le film est un chitosane ayant une masse molaire moyenne supérieure à 60 000 g.mol⁻¹, par exemple entre 60 000 g.mol⁻¹ et 200 000 g.mol⁻¹, ou bien une masse molaire comprise entre 60 000 et 150 000 g.mol⁻¹, et tout particulièrement entre 70 000 et 100 000 g.mol⁻¹. De manière préférée, le chitosane dont est constitué le film est un chitosane ayant une masse molaire moyenne supérieure à 80 000 g.mol⁻¹, et même supérieure à 100 000 g.mol⁻¹ ou 120 000 g.mol⁻¹. En effet, le chitosane étant un polymère, il existe sous différentes formes avec un degré de polymérisation qui peut varier. Le chitosane tel que préféré pour la formation d'un film selon l'invention est donc un chitosane dont la masse molaire moyenne est relativement faible par rapport aux différentes masses molaires que l'on peut trouver pour du chitosane. Pour la formation de l'hydrogel, on préfèrera au contraire une masse moléculaire plus importante afin de favoriser le processus de gélification (dit « physique ») par la présence de longues chaînes macromoléculaires.

Cependant, il est à noter que le procédé de réalisation du film peut être adapté par des techniques bien connues de l'homme du métier à des chitosane de masse molaire différente. Il est notamment envisageable d'utiliser des chitosane d'origine animale présentant une masse molaire moyenne en masse (Mw) comprise entre 150 000 et 650 000 g/mol et ayant un degré d'acétylation (DA) compris entre 0 et 45%, de préférence supérieur à 2%. Pour la réalisation de l'hydrogel, on préfèrera un chitosane (dérivé ou complexe) présentant une masse molaire moyenne en masse supérieure à 300 000 g/mol et un degré d'acétylation inférieur à 15%.

Il est particulièrement avantageux cependant d'utiliser un chitosane d'origine non animale, et plus particulièrement un chitosane provenant de champignons, tant pour le film que pour l'hydrogel. Un tel chitosane présente une masse molaire moyenne viscométrique (Mv) comprise entre 40 000 et 150 000 g/mol, et de préférence d'au moins 60 000g/mol, et un degré d'acétylation compris entre 0 et 30%, de préférence entre 2 et 20%.

Concernant le chitosane, le dérivé ou le complexe de celui-ci dont est constitué l'hydrogel présent dans les compositions selon l'invention, il a de préférence une masse molaire comprise entre 60 000 et 200 000 g.mol⁻¹, et de préférence également entre 60 000 et 150 000 g.mol⁻¹, voire entre 70 000 et 100 000 g.mol⁻¹. Alternativement, il peut également être souhaitable que sa masse molaire moyenne soit supérieure à 80 000 g.mol⁻¹ et même supérieure à 100 000 ou 120 000 g.mol⁻¹.

Il est préférable qu'au moins l'un des deux, entre le film et l'hydrogel, soit à base d'un chitosane dont la masse molaire est comprise entre 60 000 et 150 000 g.mol⁻¹, et de préférence le film, ou bien les deux. Des masses molaires moyennes particulièrement appropriées sont comprises entre 60 000 et 150 000 g.mol⁻¹, voire entre 70 000 et 100 000 g.mol⁻¹. Alternativement, des masses molaires moyennes supérieures à 80 000 g.mol⁻¹ et même supérieures à 100000 ou 120000 g.mol⁻¹ peuvent également être avantageuses.

Le film à base de chitosane contenu dans les compositions de l'invention, a de préférence une densité de 2 à 130 g par m², de préférence entre 6 à 32 g par m². Un ordre de grandeur particulièrement préféré est de 6 à 25 g par m², et tout particulièrement entre 6 et 15 g.m⁻², voire entre 6 et 12 g.m⁻².

Quant à l'hydrogel selon l'invention, sa masse par unité de surface, lorsqu'il est déposé sur le film, est de préférence comprise entre 5 et 25 g.m⁻².

De plus, il est également possible de faire varier le degré d'acétylation du chitosane qui entre dans la composition du film. De préférence, le chitosane du film selon l'invention a un degré d'acétylation compris entre 2 et 30%, de préférence entre 2 et 20%, par exemple entre 5 et 15%.

Il en est de même pour le chitosane qui est à la base de l'hydrogel.

Il est à noter à ce sujet que le degré d'acétylation varie notamment en fonction de l'origine du chitosane. Il est toutefois possible, par des moyens chimiques, de modifier le degré d'acétylation d'un chitosane, et ce quelle que soit son origine.

Dans un mode de réalisation particulièrement préféré de l'invention, le degré d'acétylation du film est inférieur au degré d'acétylation de l'hydrogel.

Selon une mise en oeuvre particulière des compositions selon la présente invention, le film à base de chitosane comprend, en sus du chitosane, du β-glucan. Ce dernier est en effet bien connu dans le milieu médical, notamment pour ses propriétés immunostimulantes. De plus, il est reconnu pour augmenter la capacité de réponse du système immunitaire contre les infections virales, fongiques, parasitaires bactériennes et néoplasiques. Plus précisément, le beta 1,3 et 1,6 glucan multiplie l'activité des macrophages dans l'organisme humain et permet ainsi au système immunitaire d'être plus réactif. Par ailleurs, le beta-1,3 et 1,4 glucan extrait de la balle d'avoine (origine céréalière) est reconnu pour ses propriétés anti-adhérentielles.

De préférence, la proportion de β-glucan dans le film à base de chitosane varie entre 0 et 20% en masse, de préférence entre 5 et 15%, plus préférentiellement encore entre 5 et 10%.

Le béta-glucan entrant dans la composition du film a de préférence pour origine la balle d'avoine, c'est-à-dire une origine végétale et non animale, ce qui est avantageux pour un dispositif médical implantable, comme explicité plus haut.

De manière similaire, l'hydrogel à base de chitosane, d'un dérivé ou d'un complexe de celui-ci, comprend également du β-glucan. Les proportions de β-glucan dans l'hydrogel sont du même ordre de grandeur que celles pour le film à base de chitosane, l'origine du beta-glucan est également préférentiellement d'origine végétale.

Il peut être particulièrement avantageux que le film et l'hydrogel contiennent tous les deux du β-glucan, en proportion égale, équivalente, comparable ou très différente. La proportion de β-glucan dans le film et l'hydrogel est de préférence égale ou équivalente dans l'un et l'autre.

Le β-glucan dont il est question dans le film ou l'hydrogel peut être du β-glucan (1,3) (ou β-(1,3)-glucan), du β-glucan (1,3) (1,6), du β-glucan (1,4), ou du β-glucan (1,4) (1,6), ou bien un mélange de ceux-ci.

Il est à noter à ce sujet que lorsque le chitosane est extrait à partir de champignons, il est naturellement accompagné de β-glucan, essentiellement de β-glucan (1,3). Dans ces conditions, il est avantageux de conserver le mélange de chitosane et de β-glucan (1,3) tel qu'il est obtenu, sans séparer les deux composés. Il peut de plus être opportun ou bien de rajouter du β-glucan (1,3) afin d'augmenter la proportion de ce composé, ou bien au contraire de rajouter du β-glucan (1,4) dont les propriétés diffèrent et sont complémentaires de celles du β-glucan (1,3).

Il est également envisagé dans le cadre de la présente invention que d'autres composants entrent dans la composition des films à base de chitosane, par exemple du hyaluronate de sodium ou de la carboxyméthylcellulose. De tels composants peuvent également entrer dans la composition d'un hydrogel à base de chitosane, d'un dérivé de chitosane ou d'un complexe de chitosane.

Le choix des composants additionnels du film et/ou de l'hydrogel doit être fait en fonction de propriétés recherchées. Dans l'optique d'une utilisation des compositions de l'invention dans des dispositifs médicaux implantables, il est nécessaire de choisir des composants dont l'utilisation est autorisée à cet effet. Il est notamment essentiel de faire usage de composants ultra-purs, dépourvus de contaminations et d'endotoxines. Il en est de même pour le chitosane, ses dérivés ou complexes entrant dans la composition du film ou de l'hydrogel tels que décrits précédemment.

De plus, dans l'optique d'une utilisation de l'association du film et de l'hydrogel (avec ou sans support textile) dans le domaine des dispositifs médicaux ou chirurgicaux implantables, il est avantageux de choisir des composants pour le film et l'hydrogel, en sus du chitosane et de l'eau, qui permettent à l'association de conserver son caractère biocompatible, biorésorbable et bioassimilable par l'organisme.

De manière tout particulièrement préférée dans le cadre de la présente invention, tant l'hydrogel que le film pour les compositions décrites, sont résorbables *in vivo.* Afin d'obtenir une telle propriété, il est important qu'aucun des constituants du film et de l'hydrogel ne s'oppose au caractère résorbable du chitosane. En effet, un film ou un hydrogel constitué exclusivement de chitosane et d'eau est naturellement résorbable *in vivo* ; l'est également un film ou un hydrogel constitué de chitosane, de β-glucan et d'eau.

Dans le cas de la première composition selon l'invention, l'ensemble de l'association est donc avantageusement résorbable *in vivo.* Dans le cas de la seconde composition selon l'invention, seul le support textile n'est, éventuellement, pas résorbable *in vivo.*

Il est à noter que le temps nécessaire pour la résorption complète du film et de l'hydrogel *in vivo* ne sont pas nécessairement du même ordre de grandeur. De préférence, l'association du film et de l'hydrogel selon la présente invention se résorbera *in vivo* au bout de 2 à 90 jours ; de manière préférentielle entre 10 et 90 jours, voire entre 30 et 90 jours. Selon le type d'application envisagée, le temps de résorption peut être ajusté par l'homme du métier.

Il est important de noter que les compositions selon la présente invention peuvent être adaptées en terme de forme, d'épaisseur et/ou de dimensions en fonction des différentes applications envisageables.

Notamment, il n'est pas exclu que des couches supplémentaires soient apposées sur l'une ou l'autre des faces des compositions décrites précédemment. Il est en effet envisage d'emprisonner la couche d'hydrogel entre deux films, le second film étant également préférentiellement à base de chitosane et permettant de renforcer tant la résistance mécanique de l'ensemble, que la résistance à la biodégradation. En effet, la biodégradation est probablement plus lente sur la face film, du fait de l'aspect sec et solide du film en comparaison de l'hydrogel. Bien entendu, la présente invention n'est pas limitée à cette couche supplémentaire et d'autres couches peuvent être superposées sur les compositions de l'invention telles que déjà décrites. En fonction de l'utilisation recherchée, l'homme du métier saura le type de couche qu'il pourrait être avantageux de superposer.

Parmi les utilisations possibles, en sus de l'utilisation en chirurgie, on peut également noter différentes utilisations par exemple dans le domaine de l'emballage, particulièrement l'emballage alimentaire. Dans cette application, il peut être avantageux de recouvrir l'hydrogel à base de chitosane par une couche de cire, par exemple une cire végétale.

La présente invention concerne tout particulièrement les compositions décrites plus haut pour une utilisation en vue d'une implantation chirurgicale ou cosméto-esthétique. Les compositions selon l'invention possèdent en effet des propriétés qui les rendent particulièrement utiles dans ce genre d'application. Il est à noter de plus que le chitosane agit par biomimétisme. En effet, du fait de sa structure chimique, le chitosane constitue un leurre des milieux biologiques, car la liaison glycosidique et les résidus N-acétyl-glucosamine de la molécule de chitosane sont les deux présents dans la structure de la matrice extracellulaire de la plupart des tissus vivants. De ce fait, les compositions selon la présente invention sont particulièrement susceptibles d'une bonne intégration dans l'organisme et d'une bonne tolérance par l'organisme.

De plus, du fait que le matériau composite constitué de l'association du support textile, du film et de l'hydrogel possède deux faces aux propriétés distinctes, celui-ci est particulièrement adapté pour la réparation des tissus. Une face est destinée à éviter la pénétration cellulaire et les adhérences post-chirurgicales car elle est lisse, non poreuse et résorbable, alors que l'autre face favorise une fixation tissulaire rapide et la recolonisation cellulaire tout en servant de renfort.

La présente invention concerne également des prothèses ou implants chirurgicaux qui comprennent des compositions telles que décrites précédemment, c'est-à-dire l'association d'un film et d'un hydrogel ou l'association d'un support textile, d'un film et d'un hydrogel selon l'invention. Ces prothèses ou implants chirurgicaux sont bien entendu pour une utilisation en chirurgie, notamment mais pas uniquement dans le cadre de la reconstruction abdomino-thoracique ou pelvienne ou dans le traitement des éventrations, ou hernies. L'invention concerne la mise au point de dispositifs médicaux ou chirurgicaux implantables dans les domaines non exhaustifs suivants : chirurgie uro-gynécologique, abdominale, pariétale,...

Du fait des propriétés différentes des deux faces des compositions, et tout particulièrement dans le cas de la seconde composition, il est possible d'obtenir une dissociation des phénomènes de cicatrisation dont les applications sont très nombreuses.

En fonction de l'utilisation chirurgicale ou cosméto-esthétique envisagée, la nature du support textile et les épaisseurs du film et de l'hydrogel seront à ajuster. Concernant le support textile par exemple, il peut être avantageux de choisir un tricot et d'ajuster les propriétés du maillage, en fonction de l'utilisation envisagée. Quand l'utilisation comme prothèse pour le renforcement de tissus très sollicités est envisagée, l'épaisseur et la résistance du support textile doivent être définies en conséquence. L'homme du métier connaît bien, en fonction des utilisations prévues, les propriétés souhaitables de la prothèse, dont celles du support textile à utiliser.

De plus, l'association du film et de l'hydrogel, avec ou sans support textile, a également des applications dans le domaine des prothèses vasculaires, afin de remplacer le collagène utilisé à l'heure actuelle pour assurer l'étanchéité.

Les compositions selon l'invention, et les prothèses ou implants réalisés à partir de celles-ci, sont notamment implantables, par exemple par voie péritonéale. Il est rappelé en effet que l'association du film et de l'hydrogel selon l'invention est suffisamment forte pour permettre la manipulation et l'implantabilité de cette association ; il en est de même de l'association du support textile, du film et de l'hydrogel.

Les compositions selon l'invention, quand elles sont utilisées pour une implantation chirurgicale ou cosméto-esthétique, permettent une réduction significative des adhérences postopératoires, notamment dans le cas des adhérence péritonéales, ce qui les rend particulièrement supérieures aux implants déjà existants.

En effet, la face support textile, dépourvue d'hydrogel favorise la colonisation cellulaire, alors que l'autre face, recouverte d'hydrogel permet d'éviter les adhérences ou les brides.

La présente invention concerne également des procédés pour la fabrication des compositions mentionnées plus haut. Elle concerne notamment un procédé pour réaliser l'association entre un film et un hydrogel à base de chitosane. Cette association se caractérise par une liaison ou interaction forte entre le film et l'hydrogel, appelée accroche. Cette accroche est réalisée par la mise en oeuvre des étapes suivantes : à partir d'une solution aqueuse à base de chitosane, solubilisé par un acide faible, on réalise un film ; ce film est ensuite partiellement séché et neutralisé ; le film ainsi obtenu, partiellement séché et neutralisé, est mis en contact avec un hydrogel à base de chitosane, en cours de formation ou bien même à l'initiation de la formation.

Dans le film partiellement séché et neutralisé, il demeure encore des molécules d'eau et des fonctions amines protonées dans une proportion supérieure à celle dans le film un fois finalisé. Comme explicité précédemment, un film partiellement neutralisé comprend au moins 30% des groupements amine du chitosane sous forme protonée. De préférence, au moins 50%, voire au moins 60%, 70 ou 75% des groupements amine du chitosane du film sont sous forme -NH₃⁺. Un exemple de réalisation est décrit dans la partie expérimentale.

Par hydrogel en cours de formation, on entend par exemple un hydrogel avant le point de gélification. Un exemple de réalisation est également décrit dans la partie expérimentale de cette demande. Le film peut être mis en contact avec la solution qui va donner l'hydrogel avant même la formation effective de l'hydrogel.

La couche de gélification peut notamment être obtenue en déposant une solution hydroalcoolique de chitosane (d'un complexe ou d'un dérivé de celui-ci) sur un support solide, par exemple une plaque en polymère. La solution initiale en chitosane doit être choisie suffisamment visqueuse pour permettre le procédé de gélification physique du chitosane, c'est-à-dire que la concentration doit être supérieure ou égale à la concentration critique d'enchevêtrement. Si le chitosane utilisé est de haute masse moléculaire (supérieure à 500 000 g/mol), alors la concentration doit être supérieure à 1,5%, elle doit être d'au moins 3%, voire d'au moins 4% pour des chitosanes dont la masse molaire moyenne est plus faible (par exemple entre 60 000 et 70 000 g/mol).

Il est à noter que ce procédé peut comprendre des étapes antérieures et postérieures à celles décrites, ainsi que des étapes intermédiaires. Il n'est donc pas limité aux étapes mentionnées.

La présente invention concerne aussi un procédé de réalisation d'un matériau comprenant un support textile recouvert d'un hydrogel à base de chitosane sur uniquement l'une de ses deux faces. Un tel procédé selon l'invention comprend les étapes suivantes : le dépôt du support textile choisi sur une surface de préférence plane ; l'étalage sur le support d'un film à partir d'une solution aqueuse à base de chitosane solubilisé par un acide faible ; le film ainsi réalisé est partiellement séché et partiellement neutralisé comme décrit précédemment ; enfin l'hydrogel en formation est mis en contact avec la face du film qui est opposée à celle du support.

Des étapes postérieures, antérieures et / ou intermédiaires sont bien entendu envisageables.

Selon les procédés de l'invention, le chitosane est solubilisé dans un acide faible, de préférence un acide faible permettant de réaliser simultanément la neutralisation du film et l'évaporation de l'eau (séchage). Les acides faibles particulièrement préférés sont ceux dont la température d'évaporation (à la pression atmosphérique) est basse, par exemple inférieure à 130°C, de préférence inférieure à 120°C. Un acide faible tout particulièrement préféré dans le cadre de la présente invention pour solubiliser le chitosane (ou un dérivé ou un complexe de celui-ci) est l'acide acétique. Du fait que la température d'ébullition de l'acide acétique est basse (118°C), cela permet un départ spontané de l'acide acétique en présence d'eau même à température ambiante.

Selon le procédé décrit, le support textile est de préférence tendu sur une surface plane afin d'être lui-même parfaitement plan.

La présente invention concerne également l'utilisation d'un film à base de chitosane. En effet, un tel film est avantageux pour réaliser une séparation entre un support d'une part et un hydrogel d'autre part. Un film à base de chitosane peut donc être utilisé pour réaliser une composition, ou matériau composite, comprenant un support textile recouvert d'un hydrogel à base de chitosane sur uniquement l'une de ses deux faces, ledit film assurant le rôle de barrière entre le support textile et l'hydrogel afin que l'hydrogel ne recouvre pas les deux faces du support textile.

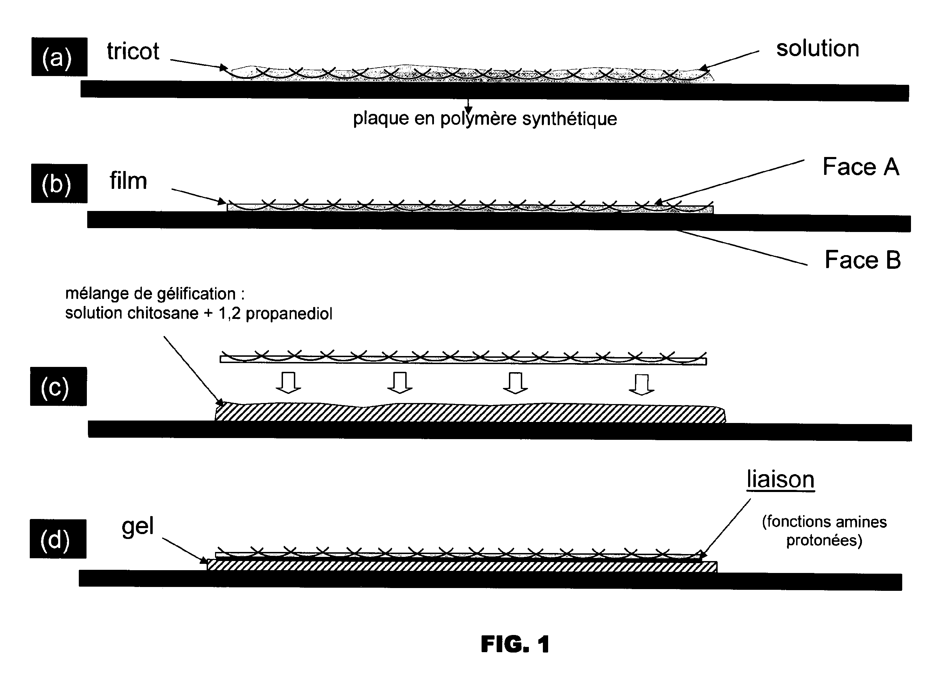

**Figure 1** : Cette figure illustre un mode de réalisation d'une composition comprenant un textile, un film et un hydrogel.

### EXEMPLES

**Exemple 1** : Réalisation d'une prothèse composite formée d'un support textile, d'un film à base de chitosane et d'un hygrogel à base de chitosane.

### Introduction :

La prothèse réalisée par les présents inventeurs est dédiée à une utilisation dans le domaine des dispositifs médicaux ou chirurgicaux implantables, pour la prévention des adhérences post-chirurgicales.

La prothèse décrite est préférentiellement destinée au renfort des insuffisances pariétales (déficit de la paroi dans le cas des éventrations) et adaptée au traitement intra-péritonéal en présentant une propriété anti-adhérence. L'implantation de cette prothèse par voie intra-péritonéale permet de compenser la défaillance du péritoine, d'obstruer l'orifice herniaire et de limiter au maximum le phénomène d'adhérences entre le renfort et les viscères.

Cette prothèse est réalisée afin d'obtenir un textile composite comprenant deux faces distinctes aux propriétés différentes, grâce à l'utilisation d'une surface anti-adhérentielle. La prothèse sert donc de barrière tant physique que mécanique, afin d'empêcher l'adhésion des tissus au cours de la cicatrisation.Toutefois, une fois que la cicatrisation est terminée, et donc que les risques d'adhésion sont inexistants, cette barrière physique doit disparaître sans nécessiter de nouvelle intervention chirurgicale pour la retirer, et seul demeure le support textile.

La prothèse textile est composée de 3 couches successives permettant d'obtenir deux faces distinctes aux propriétés suivantes :
- 1 face dite « rugueuse », non résorbable, (textile)

Cette face est à implanter du côté de la paroi ou des tissus biologiques afin de permettre l'« accroche mécanique » et le rôle de renfort et maintien des tissus, elle permet également la réhabitation tissulaire; le textile est de préférence un treillis en monofilaments ou multifilaments de polymère synthétique non résorbable, tel que du polypropylène ou du polyester ;
- 1 face dite « lisse » biorésorbable, (hydrogel à base de chitosane)

Cette face permet de limiter les phénomènes d'adhérences et facilite l'intégration de la prothèse en réduisant la réaction inflammatoire et en facilitant le phénomène de cicatrisation.

Les 2 faces peuvent être distinguées sans difficultés à l'oeil nu.

Les inventeurs ont élaboré un nouveau procédé pour réaliser la liaison entre le tricot et l'hydrogel à base de chitosane, contenant ou non du beta-glucan (à l'état naturel ou ajouté).

Ce procédé nouveau dit « de liaison entre tricot/film et film/hydrogel » permet l'élaboration d'un matériau textile composite, comprenant un tissu tricoté (de polypropylène ou polyester) associé à un film de chitosane résorbable *in vivo,* et à un hydrogel de chitosane.

Le procédé d'obtention de ce « textile composite » peut se décliner en 2 étapes :
1) enduction du textile d'un film de chitosane ;
2) puis accroche de l'hydrogel de chitosane sur le film, afin de pouvoir distinguer les 2 faces.

Ce mode de liaison permet de renforcer la fixation de l'hydrogel sur le tricot. De plus, puisqu'un gel peut difficilement enduire de façon homogène et régulière un textile, la recolonisation par les cellules se fera donc de façon irrégulière. En enduisant préalablement le textile d'un film de chitosane, on peut 'niveler' la surface de la prothèse et permettre une recolonisation plus propre, plus nette et plus uniforme, ce qui est d'une grande importance pour un implant chirurgical.

Le textile utilisé doit être, de préférence, un tricot à larges pores, en monofilaments de polypropylène, de faible épaisseur et de faible grammage, non tridimensionnel.

Plusieurs procédés de gélification du chitosane peuvent être adaptés à ce procédé de liaison.

Les inventeurs ont envisagé trois solutions de procédés de gélification :
- système de gélification physique du chitosane par voie gazeuse (ammoniac) ;
- utilisation d'un agent complexant naturel : le xanthane pour permettre la gélification du chitosane ;
- gélification physique du chitosane en milieu hydro-alcoolique, avec ou sans étape de neutralisation par une base.

Le but est de permettre la gélification du chitosane in-situ sur le treillis en polymère synthétique. Cependant, la description des procédés de gélification possibles se limitera dans le cadre de cet exemple au procédé de gélification par voie hydro-alcoolique.

Le procédé peut être adapté à toutes les sortes de chitosane :
- d'origine animale (carapaces de crustacés, endosquelettes de calamars) :
   ce chitosane présente une masse molaire moyenne en masse (MW) comprise entre 150 000 et 650 000 g/mol, et un degré d'acétylation (DA) compris entre 0 et 20 %, de préférence > 2 % ;
- ou d'origine non-animale (champignons)
   ce chitosane présente une masse molaire moyenne viscosimétrique (Mv) comprise entre 40 000 et 150 000 g/mol, de préférence supérieure à 60 000 g/mol, et un DA compris entre 0 et 30 %, de préférence entre 2 et 20 %.

### Procédé d'élaboration de la prothèse composite en 3 étapes (voir description du procédé par 4 schémas (a), (b), (c) et (d), Figure 1)

Le procédé décrit doit bien sûr être adapté en fonction des propriétés du textile utilisé et des masses molaires moyennes des produits à base de chitosane utilisés.

Dans ce qui suit, le procédé est plus particulièrement décrit avec un tricot dont les principales caractéristiques sont les suivantes : tricot en monofilaments de polypropylène de 0,08 mm de diamètre ; grammage de 22 g/m² ; épaisseur de 0,28 mm ; composé de pores de taille différente, mais la surface occupée par les plus grands pores est d'au moins 0,5 mm² (environ 0,6 mm²).

### A) Enduction du tricot avec la « 1^{ère} couche : FILM »

Etape 1). Il s'agit tout d'abord de dissoudre le chitosane (Glc-NH₂) dans une solution aqueuse d'acide acétique, l'acide acétique étant ajouté en quantité stoechiométrique par rapport aux fonctions amines (voir plus bas, réaction A). La concentration en chitosane étant comprise entre 1 et 5% (w/w) selon Mw initiale. Après dissolution complète du chitosane (entre 3 h et 1 nuit d'agitation, magnétique ou mécanique, selon les échantillons et la concentration de la solution en polymère), la solution peut être éventuellement filtrée pour éliminer les insolubles et toutes particules qui ne seraient pas biorésorbables (filtres de porosité comprise entre 0,45 et 1 µm).
Etape 2).

### [schéma (a) figure 1]

Ensuite, la solution est étalée sur le tricot (non tridimensionnel, et de préférence à larges pores [> 200 µm dans une des dimensions]) dans une proportion comprise entre 25 et 75 mg/cm² de textile, qui est lui-même fixé sur une plaque en polymère synthétique, de préférence en polystyrène, présentant un état de surface lisse et sans défaut apparent. La concentration de la solution doit être suffisamment élevée pour présenter une viscosité qui permet d'étaler la solution sans difficulté et de combler tous les pores de la maille du tricot. La quantité de solution étalée dépend de la concentration de la solution initiale.

Dans un mode de réalisation avantageux, la masse de solution ajoutée est comprise entre 35 et 45 mg/cm² de textile.
Etape 3). Ensuite, il est nécessaire de sécher la solution, en présence ou non d'air chaud pendant 1 nuit, afin d'extraire l'eau de la solution et obtenir ainsi un film de chitosane. En effet, le film va alors se former au contact de cette plaque en polymère et adhérer au tricot sur une faible épaisseur (le tricot reste à la surface du film et le film se trouve contre la plaque en polymère).

### [schéma (b) figure 1]

Il est important de préciser qu'à cette étape du procédé, on est en présence d'un premier composite où l'on distingue déjà 2 faces au toucher: une face tricot (Face A [schéma (b)] figure 1) et une face film (qui est en contact avec la plaque de polymère synthétique, Face B [schéma (b)] figure 1).

De plus, la face film est lisse, régulière et sans défaut, puisqu'elle s'est formée au contact de la plaque polymère solide, lisse, régulière et sans défaut. Le textile est en quelque sorte noyé ou emprisonné dans la 1^{ère} matrice 'film' mais sur une faible épaisseur puisque le film ressort en relief, côté atmosphère ambiante.

Le film est alors composé d'un sel de chitosane : l'acétate de chitosane (GLc-NH₃⁺,CH₃COO⁻), soluble dans l'eau dans les proportions suivantes :
- chitosane (en association avec entre 0 et 10 % de beta-glucan (1,3) à l'état naturel quand le chitosane provient de champignons) dans une proportion comprise entre 80 et 90 %, telle que la masse de chitosane (et de beta-glucan naturellement associé) ajoutée par unité de surface du textile soit comprise entre 2,5 et 16 g/m², de préférence entre 6 et 12 g/m² ;
- entre 0 et 10 % de beta-glucan (1,4) ;
- entre 3 et 20 % d'eau.

### B) Accroche de la « 2^{ème} couche : GEL» sur le tricot enduit du film

### Etape 4) [schéma (b) figure 1]

Pour permettre l'accroche de l'hydrogel de chitosane sur la face film du tricot, le film doit être neutralisé partiellement (donc partiellement soluble dans l'eau). Il s'agit d'un état « mi-neutre », intermédiaire : mi-chitosane / mi-acétate de chitosane qui peut être obtenu par une étape dite de « POST-SECHAGE » sur le film.

Plus précisément, l'étape de « POST-SECHAGE » consiste à contrôler l'hydrolyse de l'acide acétique dans des conditions atmosphériques ambiantes (22°C, 50 % humidité relative ou RH) ou dans des conditions contrôlées en température et en humidité (40°C, 55 % RH).

En effet, l'acide acétique, acide faible, est facilement soluble dans l'eau et possède un point d'ébullition faible (118°C). On observe alors un phénomène de départ spontané de l'acide, qui est accentué en présence d'une quantité plus importante d'eau (réaction B). De plus, l'acide acétique étant une entité très volatile, s'éliminera plus facilement en présence de chaleur et de ventilation. Le phénomène peut se décrire selon la réaction suivante (réaction B) :

Réaction A Glc+NH₂₍ₛ₎+CH₃COOH_{(aq)}+H₂O→GLc-NH₃⁺_{(aq)}+CH₃COO⁻_{(aq)}+H₂O

Réaction B (GLc-NH₃⁺,CH₃COO⁻)₍ₛₐₗₜ₎+H₂O⇄Glc+NH₂₍ₛ₎+CH₃COOH_{(gaz)}+H₂O

Il s'agit donc de stopper la réaction au bout d'un certain temps pour obtenir un film comportant les 2 formes de chitosane :

(GLc-NH₃⁺,CH₃COO⁻)₍ₛₑₗ₎+H₂O→Glc+NH₂₍ₛ₎+CH₃COOH_{(gaz)}+(GLc-NH₃⁺,CH₃COO⁻)₍ₛₑₗ₎+H₂O

En fait, le but du post-séchage est d'obtenir une proportion en acétate de chitosane dans le film d'environ 60 % (entre 50 et 70 %) au moment de le déposer sur la couche gel. Il faut donc un temps de post-séchage qui se situe entre 1 et 2 jours à atmosphère ambiante (valable pour un DA entre 2 et 20 %). Le temps de post-séchage est bien sûr à adapter en fonction du DA.

La proportion finale est telle que la quantité de fonctions amines protonées dans le « film pas complètement neutralisé », ou le « film mi-neutre » est d'environ 60 % (ceci représentant une moyenne, avec par exemple une variation d'une face à l'autre du film de 30 % à 90 % de fonctions amine protonée).

Dans un souci d'industrialisation, on peut cependant réduire ce temps de « post-séchage » dans une atmosphère humide (60 % HR) et chaude (40°C), et de préférence, ventilée (enceinte climatique) afin d'accélérer l'hydrolyse partielle de l'acide acétique (quelques heures suffiraient à l'hydrolyse du sel (acétate de chitosane) en surface).

Il est essentiel que l'hydrolyse soit partielle car les fonctions amines qui resteront protonées permettent la liaison entre le film et l'hydrogel de chitosane. Dans le cas où le film serait complètement neutralisé, donc insoluble dans l'eau, il serait difficile voire impossible de former l'accroche entre l'hydrogel et le film.

En effet, si la proportion en acétate de chitosane est inférieure à 30 % (soit un temps de post-séchage supérieur à 1 jour en milieu humide ou supérieur à 3 jours à atmosphère ambiante), on risque de ne plus avoir le bénéfice d'une liaison possible entre les 2 entités 'film' et 'hydrogel', le mélange hydroalcoolique aurait tendance à glisser contre le film sec sans pouvoir trouver d' « accroche ».

Enfin, il est à noter qu'il est important de maintenir le tricot tendu lors du séchage du film pour éviter des déformations qui engendrent par la suite des défauts de structure dans le film.

### Exemples de préparation de cette 1^{ère} couche 'FILM':

Pour un chitosane d'origine végétale de Mv = 70 000 g/mol et DA = 16 %
- concentration de la solution initiale : 3% (w/w)
- masse de la solution ajoutée par unité de surface de textile : 40,8 mg/cm²
- quantité de chitosane ajoutée par unité de surface de textile : 12,2 g/m²
   → temps de séchage : 1 nuit
   → + temps de 'post-séchage' pour la « demi-neutralisation » : entre 1 et 2 jours à atmosphère ambiante

Pour un chitosane d'origine animale de Mw > 500 000 g/mol (650 000 g/mol) et d'un DA = 5%
- concentration de la solution initiale : 1,5 % (w/w)
- masse de la solution ajoutée par unité de surface de textile : 40,8 mg/cm²
- quantité de chitosane ajoutée par unité de surface de textile : 6,1 g/m²
   → temps de séchage : 1 nuit
   → + temps de 'post-séchage' pour la « demi-neutralisation » : entre 1 et 2 jours à atmosphère ambiante

Etape 5). Dans le même temps, on prépare le mélange composé d'une solution concentrée de chitosane et de 1,2-propanediol qui va permettre de former la couche « GEL » du composite.

Il s'agit d'obtenir un hydrogel physique de chitosane par évaporation en milieu hydro-alcoolique, selon le protocole expérimental suivant :
Tout d'abord, le chitosane est dissout dans une solution d'acide acétique ; l'acide acétique étant ajouté en quantité stoechiométrique par rapport aux fonctions amines. La concentration de la solution doit être suffisamment élevée pour permettre l'enchevêtrement des chaînes macromoléculaires jusqu'à la percolation, rendant compte de la gélification du chitosane.
La concentration doit être supérieure ou égale à la concentration critique d'enchevêtrement, elle doit donc être supérieure à 2,5 % pour un chitosane de haute masse molaire (> 500 000 g/mol) et doit être au moins de 3 %, de préférence > 4% pour des plus faibles masses molaires moyennes (entre 60 000 et 70 000 g/mol).
Après dissolution complète, on peut envisager d'ajouter du beta-1,4-glucan pour augmenter la proportion de beta-glucan dans l'hydrogel.
Puis, après avoir éventuellement filtré la solution concentrée de chitosane sur des filtres de porosité comprise entre 0,45 et 1 µm de diamètre, on ajoute un volume (ou masse) équivalent de 1,2-propanediol (composition du mélange de chitosane hydroalcoolique : environ 50 % de solution de chitosane et 50 % de 1,2-propanediol (en masse)). Le mélange est agité fortement pendant environ une demi-heure, puis est dégazé à atmosphère ambiante (ou sous pression réduite, si nécessaire : dans le cas où la solution serait très visqueuse).

### [schéma (c) figure 1]

On étale alors une certaine quantité du mélange de chitosane hydroalcoolique de gélification (composé d'une solution concentrée de chitosane et du 1,2-propanediol), comprise entre 30 et 150 mg/cm², et de préférence entre 50 et 100 mg / cm² (de textile) sur la plaque en polymère synthétique selon l'épaisseur finale que l'on souhaite atteindre pour le gel (cette épaisseur peut aussi être influencée par le temps de réaction, qui correspond à l'évaporation de l'eau du mélange).

Selon la concentration de la solution de chitosane initiale, la masse de chitosane qui sera ajoutée à l'association du tricot et du film sera alors comprise entre 5 et 25 g/m², de préférence entre 10 et 15 g/m².

STABILISATION du mélange hydro-alcoolique sur la plaque : amorçage de la réaction de gélification. Cette étape n'est pas obligatoire. On peut mettre le film (ou le film + textile) avant même l'amorçage de la réaction de gélification.

Avant d'atteindre le point de gélification, on pose alors l'association du tricot et du film, préalablement décollé de la plaque en polymère, face B contre le mélange de chitosane hydroalcoolique de gélification qui se trouve sur la plaque en polymère. Le tricot est ainsi attiré par le gel sans pénétrer à travers. On peut observer que le tricot reste en surface grâce à la barrière physique qui a été constituée par le film.

Par ailleurs, l'accroche est très facile puisque le film, n'étant pas complètement neutralisé sur sa face B, comporte encore une forte proportion de fonctions amines protonées en surface ce qui permet de former une petite « COUCHE DE LIAISON », ce qui autorise l'accroche entre ces dernières et les autres charges négatives, notamment, présentes dans le mélange hydroalcoolique (acide acétique, chitosane, eau, 1,2 propanediol) du gel en formation et ainsi permettre la liaison par un phénomène de 'ressolubilisation' en surface.

La face A, avec une plus forte proportion en chitosane, sous sa forme neutre, permettra de remplir sa fonction barrière en maintenant et préservant la forme solide du film. Ainsi, le mélange hydroalcoolique ne traversera pas les mailles du tricot et ressortira en relief, côté face B du film.

### C) Gélification puis neutralisation : obtention de la prothèse COMPOSITE

### Etape 6) [schéma (d) figure 1]

L'ensemble prothèse composite comportant le mélange de gélification est ainsi laissé au repos, à atmosphère ambiante (22°C, 50 % RH), pendant un temps donné (qui varie selon la concentration initiale en polymère, les conditions de température et de pression, et la concentration finale que l'on souhaite atteindre) afin de favoriser l'évaporation de l'eau et permettre ainsi la gélification.

Les temps de réaction à atmosphère ambiante sont alors les suivants (pour une MW de 70 000 g/mol) :
- pour 35 mg du mélange chitosane + 1,2-propanediol pour une surface de 1 cm² (contenant une solution à 3 ou 4% en masse de chitosane) : 4 jours sont suffisants (si on attend plus longtemps, le mélange sèche) ;
- pour 60 mg du mélange / cm² (contenant une solution à 3 ou 4% en masse de chitosane) : 5 jours de réaction ;
- entre 80 et 125 mg de mélange / cm² : 7 jours de réaction.

### Exemples de réalisation de la couche 'hydrogel' :

Pour un chitosane d'origine végétale de Mv = 70 000 g/mol et DA = 16 % :
- concentration de la solution initiale : 4% (w/w),
- masse de la solution ajoutée par unité de surface de textile : 61 mg/cm²,
- quantité de chitosane ajoutée par unité de surface de textile : 12,2 g/m²,
   → temps de réaction pour le processus de gélification : 5 jours à atmosphère ambiante.

Pour un chitosane d'origine animale de Mw > 500 000 g/mol (650 000 g/mol) et d'un DA = 5% :
- concentration de la solution initiale : 2,5 % (w/w),
- masse de la solution ajoutée par unité de surface de textile : 82 mg/cm²,
- quantité de chitosane ajoutée par unité de surface de textile : 10,2 g/m²,
   → temps de réaction pour le processus de gélification : 8 jours à atmosphère ambiante.

Ces résultats sont donnés à titre indicatif, mais, de la même façon que pour le film, ces temps de réaction pourraient être raccourcis si on se place dans des conditions de température et d'humidité optimales.

Après ce temps de réaction, le gel obtenu est neutralisé avec une solution de soude 0,1 mol/L ou d'ammoniaque 0,1 mol/L afin de régénérer toutes les fonctions amine et est lavé à l'eau déminéralisée (au minimum 10 lavages) pour supprimer l'alcool présent dans le gel et retrouver le pH neutre.

Un autre procédé de neutralisation des gels à l'alcool peut se faire sans agents chimiques, dans des conditions de température et d'humidité plus élevés (40°C et 60% RH minimum) directement. Ce procédé est basé sur le phénomène de départ spontané de l'acide, qui est accentué en présence d'une quantité plus importante d'eau, comme dans le procédé d'élaboration simplifié du film.

### Caractéristiques du produit final :

La prothèse textile composite (ou 'multi-couches') ainsi réalisée est donc composée de :
- un **textile,** de préférence un tricot, à larges pores et constitué de monofilaments de polypropylène et non-tridimensionnel,
- un **film constitué** de :
   * chitosane dans une proportion comprise entre 80 et 90 % (comprenant en outre entre 0 et 10 % de beta-glucan (1,3) à l'état naturel), telle que la masse de chitosane (associé au beta-glucan (1,3) à l'état naturel) ajoutée par unité de surface du textile : entre 2,5 et 16 g/m², de préférence entre 6 et 12 g/m²
   * entre 0 et 10 % de beta-glucan (1,4)
   * entre 3 et 20 % d'eau,
- un **hydrogel** constitué de :
   * chitosane dans une proportion inférieure ou égale à 5% (comprenant en outre entre 0 et 10 % de beta-glucan (1,3) à l'état naturel), telle que la masse de chitosane (associé au beta-glucan (1,3) à l'état naturel) ajoutée par unité de surface du textile : entre 5 et 25 g/m², de préférence entre 10 et 15 g/m²
   * entre 0 et 10 % de beta-glucan (1,4)
   * au moins 95 % d'eau.

L'épaisseur cumulée du film et du gel ajouté est de préférence inférieure à 2 mm, de préférence comprise entre 0,5 et 1,5 mm.

La quantité de chitosane ajouté par prothèse composite (cumul du film et de l'hydrogel) est comprise environ entre 15 et 40 g/m².

En choisissant un chitosane provenant de champignons, la masse molaire moyenne en masse (Mw) se situe entre 40 000 et 100 000 g /mol, on préfère cependant une Mw comprise entre 70 000 et 100 000 g/mol.

Contrairement aux carapaces de crustacés qui présentent des masses molaires moyennes en masse beaucoup plus élevées, on peut dans notre cas obtenir des solutions à concentrations élevées, sans pour autant obtenir des viscosités trop importantes rendant la solution inexploitable.

On obtient alors des films puis des gels de plus ou moins grosses épaisseurs selon la quantité et la concentration de la solution initiale

### Autre caractéristique de cette prothèse textile composite

Cette prothèse ainsi réalisée a de plus la propriété d'être très fine, ce qui permet de n'implanter que très peu de matériau étranger lors de l'utilisation chirurgicale.

De plus, le textile garde une certaine souplesse malgré la présence d'un film et d'un gel, il est donc aisément manipulable et permet de s'adapter in vivo lorsqu'il est implanté.

**Exemple 2 :** Principales étapes d'un mode de réalisation d'une prothèse composite formée d'un support textile, d'un film à base de chitosane et d'un hygrogel à base de chitosane.

Le textile utilisé dans cet exemple illustratif est le même tricot que celui décrit dans l'exemple 1.
**(a)** Après avoir solubilisé le chitosane dans une solution d'acide acétique, la solution est étalée sur un tricot qui est lui-même fixé sur une plaque en polymère synthétique (lisse, régulier, sans défaut de surface) ;
**(b)** Après 1 nuit à l'air ambiant, on laisse le tricot sous une ventilation « humide » de préférence (étape de « POST-SECHAGE ») pour permettre l'hydrolyse partielle de l'acide acétique, en particulier sur la Face A (entre 1 et 2 jours à l'air ambiant, selon le DA du chitosane).
**(c)** Après avoir préparé le mélange de gélification constitué d'une solution concentrée de chitosane et d'un volume équivalent de 1,2-propanediol, on décolle le tricot+film et on étale une certaine quantité du mélange.
   On pose alors le tricot+film, face B sur le mélange de gélification, la face B comportant encore une forte proportion de fonctions amines protonées
**(d)** Le film est attiré par le mélange (grâce à la présence de charges négatives notamment) et se colle sur ce dernier en formant une barrière qui permet au tricot de rester à la surface.
   Après au moins 4 jours de « réaction » à atmosphère ambiante (influence de la concentration et des paramètres physico-chimiques du chitosane sur le temps de gélification), on neutralise le composite avec une solution de soude 0,1 molaire et on effectue une dizaine de lavage pour retrouver le pH neutre et retirer le 1,2-propanediol.

### RESULTAT : prothèse TEXTILE COMPOSITE comportant 2 faces bien distinctes :

- une face tricot qui va permettre la réhabitation de la prothèse ds les tissus de l'organisme par sa structure ;
- une face biorésorbable sous forme de gel.

La face gel est lisse et régulière puisqu'elle s'est formée en contact d'une plaque en polymère synthétique lisse et homogène.

Le film a joué le rôle d'une barrière PHYSIQUE et permet ainsi d'avoir 2 couches bien distinctes.

Il est à noter que les temps de réaction peuvent être optimisés (et raccourcis) dans des conditions d'humidité et de température plus élevées (ex : 40°C et 60% HR).

La prothèse composite présente alors les caractéristiques avantageuses suivantes :
- un véritable gel physique ayant une épaisseur inférieure à 2 mm,
- Mw compris entre 60 000 et 600 000 g/mol,
- DA compris 2 et 20%,
- Nouveau mode de liaison entre le textile et le gel de chitosane ,
- Textile utilisé : tricot de faible épaisseur (de préférence en monofilaments de polyprolpylène), de préférence, utilisation d'un tricot « dit light » pour éviter ainsi d'implanter des quantités importantes de polymère synthétique (réduction supplémentaire de la réaction inflammatoire),
- Chitosane associé ou non à du beta-glucan .

## Revendications

1. Composition comprenant l'association:
- d'un film à base de chitosane, comprenant moins de 20% d'eau (en masse), et
- d'un hydrogel à base de chitosane, d'un dérivé de chitosane ou d'un complexe de chitosane, comprenant au moins 80% d'eau (en masse).

2. Composition comprenant l'association:
- d'un support textile;
- d'un film à base de chitosane, comprenant moins de 20% d'eau (en masse) et
- d'un hydrogel à base de chitosane, d'un dérivé de chitosane ou d'un complexe de chitosane, comprenant au moins 80% d'eau (en masse),
tel que l'hydrogel ne soit présent que sur l'une des deux faces du support textile.

3. Composition selon la revendication 2, **caractérisé en ce que** ledit support est un support textile tissé, non-tissé ou bien tricoté.

4. Composition selon la revendication 3, **caractérisé en ce que** ledit textile est un tricot à larges pores, en polymère, de préférence en polymère synthétique, de préférence en monofilaments de polypropylène.

5. Composition selon la revendication 2, tel que le film est déposé sur le support textile par enduction du support avec une solution à base de chitosane.

6. Composition selon la revendication 1 ou 2, tel que le film a une épaisseur inférieure à 0.15 mm, de préférence inférieure à 0.03 mm.

7. Composition selon la revendication 1 ou 2, tel que l'accroche permettant l'association entre le film et l'hydrogel est réalisée par mise en contact d'un film non complètement neutralisé avec l'hydrogel en cours de formation.

8. Composition selon la revendication 7, tel que le film non complètement neutralisé est un film comprenant au moins 30% des groupements amine du chitosane sous forme protonéee, de préférence au moins 50% ou au moins 60%.

9. Composition selon la revendication 1 ou 2, **caractérisé en ce que** le chitosane à la base du film et/ou de l'hydrogel est un chitosane ayant une masse molaire entre 60 000 et 150 000 g.mol⁻¹.

10. Composition selon la revendication 1 ou 2, **caractérisé en ce que** le chitosane à la base du film et/ou de l'hydrogel est un chitosane d'origine non animale, de préférence extrait de champignons.

11. Composition selon la revendication 1 ou 2, **caractérisé en ce que** le film et/ou l'hydrogel à base de chitosane comprennent également du β-glucan, en proportion variant entre 0 et 20% en masse par rapport au chitosane.

12. Composition selon la revendication 11, tel que le β-glucan est du β-glucan (1,3) et/ou du β-glucan (1,4).

13. Composition selon la revendication 1 ou 2, **caractérisé en ce que** l'hydrogel et le film sont résorbables *in vivo.*

14. Prothèse pour une utilisation chirurgicale comprenant une composition selon l'une quelconque des revendications 1 à 13.

15. Procédé d'association entre un film et un hydrogel à base de chitosane, **caractérisé en ce que** l'accroche permettant l'association comprend les étapes suivantes :
- réalisation d'un film à partir d'une solution aqueuse à base de chitosane solubilisé par un acide faible ;
- séchage et neutralisation partiels du film ;
- mise en contact du film et de l'hydrogel en formation.

16. Procédé de réalisation d'un matériau comprenant un support textile recouvert d'un hydrogel à base de chitosane sur uniquement l'une de ses deux faces, comprenant les étapes suivantes :
- dépôt du support textile sur une surface plane ;
- étalage sur le support d'un film à partir d'une solution aqueuse à base de chitosane solubilisé par un acide faible ;
- séchage et neutralisation partiels du film ;
- mise en contact dudit hydrogel en formation avec la face du film opposée à celle du support.

17. Utilisation d'un film à base de chitosane, pour réaliser une composition comprenant un support textile recouvert d'un hydrogel à base de chitosane sur uniquement l'une de ses deux faces, ledit film assurant le rôle de barrière entre le support textile et l'hydrogel afin que l'hydrogel ne recouvre pas les deux faces du support textile.

18. Composition selon l'une quelconque des revendications 1 à 13 pour une utilisation en vue d'une implantation chirurgicale ou cosméto-esthétique.
